# EUROPEAN PATENT APPLICATION

(11) **EP 2 071 015 A2**
(43) Date of publication of application: **17.06.2009**
(21) Application number: 08251431.6
(22) Date of filing: 15.04.2008
(51) Int. Cl.: C11C 3/00, C07C 41/05, C10L 1/02

(54) **Method for producing stabilized biodiesel fuel**

(30) Priority: 14.12.2007 US 2182
(71) Applicant: Rohm and Haas Company, Philadelphia, PA 19106-2399 (US)
(72) Inventor: Banavali, Rajiv Manohar, Rydal, Pennsylvania 19046 (US); Schultz, Alfred Karl, Maple Glen, Pennsylvania 19002 (US); Hsu, Shih-Ying, Chalfont, Pennsylvania 18914 (US)
(74) Representative: Kent, Venetia Katherine

(57) **Abstract**

A method for production of stabilized fatty acid methyl esters. The method removes unsaturation present in fatty acid methyl esters, or removes unsaturation in triglycerides, which are then transesterified to fatty acid methyl esters.

## Description

### Background

This invention relates generally to a method for producing stabilized biodiesel fuel.

High fuel prices and environmental concerns are driving development of alternative fuels, especially those derived from renewable resources. One such fuel, commonly known as "biodiesel" fuel, contains alkyl, usually methyl esters of fatty acids, and is burned in diesel engines. Biodiesel fuel typically is produced from transesterification of triglycerides, such as vegetable oils, with methanol. However, due to the nature of the unsaturated fatty acids in triglycerides, the resulting fuel has a high degree of oxidative instability, making it unsuitable for long-term storage and handling. For example, G. Knothe, Fuel Processing Technology, vol. 88, pp. 669-677 (2007) discloses addition of antioxidants to stabilize biodiesel fuels. However, this reference points out that the antioxidants do not prevent oxidation, but merely delay it, and thus alternative methods for stabilizing biodiesel fuels would be desirable.

The problem addressed by this invention is to find an alternative method for producing stabilized biodiesel fuel.

### Statement of Invention

The present invention is directed to a method for production of stabilized fatty acid methyl esters; said method comprising either: (a) (i) contacting an unsaturated triglyceride with a C₁-C₄ alcohol and an acidic catalyst to produce a triglyceride ether, and (ii) transesterifying said triglyceride ether with methanol to produce a stabilized fatty acid methyl ester; or (b) contacting an unsaturated fatty acid methyl ester with a C₁-C₄ alcohol and an acidic catalyst to produce a stabilized fatty acid methyl ester.

### Detailed Description

All percentages are weight percentages (wt%), and all temperatures are in °C, unless otherwise indicated. Weight percentages of ion exchange resin are based on dry resin. An "alkyl" group is a saturated hydrocarbyl group having from one to twenty-two carbon atoms in a linear or branched arrangement. "Triglycerides" used in this invention are fats or oils comprising glycerine triesters of fatty acids. Triglycerides may be in the form of vegetable oils and/or animal fats. Triglycerides also may contain free fatty acids. Fatty acids are acyclic aliphatic carboxylic acids containing from 8 to 22 carbon atoms; typically, they contain from 12 to 22 carbon atoms. With respect to carbon-carbon bonds, the fatty acids may be saturated, monounsaturated or polyunsaturated (typically 2 or 3 carbon-carbon double bonds). Natural fats may also contain small amounts of other esterified, or free fatty acids, as well as small amounts (1-4%) of phospholipids, e.g., lecithin, and very small amounts (<1%) of other compounds, e.g., tocopherols. "Unsaturated triglycerides" or "unsaturated fatty acid methyl esters" are those having at least one unsaturated fatty acid group.

The acidic catalyst is believed to facilitate addition of the C₁-C₄ alcohol across an unsaturated carbon-carbon bond to produce a C₁-C₄ alkyl ether of the fatty acid methyl ester or triglyceride. Any acidic catalyst known to facilitate addition of alcohols to carbon-carbon double bonds may be used for the alcohol addition reaction. In some embodiments of the invention, the acidic catalyst is an acidic ion exchange resin. In some embodiments of the invention, the acid functionality of the ion exchange resin comprises sulfonic acid groups, carboxylic acid groups, phosphoric acid groups or a mixture thereof. In some embodiments of the invention, an acidic ion exchange resin used to facilitate alcohol addition in the present invention is a gel-type resin, not a macroreticular resin. A macroreticular resin is a resin having a surface area from 25 m²/g to 200 m²/g and an average pore diameter from 50 Å to 500 Å; alternatively a surface area from 30 m²/g to 80 m²/g and an average pore diameter from 100 Å to 300 Å. Suitable gel-type resins include, e.g., acrylic resins, styrenic resins, and combinations thereof. Resins contain polymerized units of a multiethylenically unsaturated monomer (crosslinker). In some embodiments of the invention, the level of crosslinker in the resin is no more than 2.75%, alternatively no more than 2.5%, alternatively no more than 2.25%, alternatively no more than 2%, alternatively no more than 1.75%. In one embodiment, the level of crosslinker is at least 0.5%, alternatively at least 0.75%, alternatively at least 1%, alternatively at least 1.25%. Preferably, the average particle size of the gel resin is from 100 µm to 2000 µm, more preferably from 200 µm to 800 µm. In some embodiments of the invention, the ion exchange resin comprises polymerized units of styrene and a crosslinker, e.g., divinyl aromatics; di-, tri- and tetra-(meth)acrylates or (meth)acrylamides; di-, tri- and tetra-allyl ethers and esters; polyallyl and polyvinyl ethers of glycols and polyols. In some embodiments of the invention, the crosslinker is diethylenically unsaturated, e.g., divinylbenzene (DVB). A typical acidic ion exchange resin has from 0.4 to 8 meq/g acid functionality, on a dry basis, alternatively at least 2 meq/g, alternatively at least 4 meq/g. Preferably, the acid functionality is in the form of sulfonic acid groups. In some embodiments of the invention, the acidic catalyst is selected from zeolites, acidic silicas, acidic diatomaceous earth, and combinations thereof.

Preferably, the C₁-C₄ alcohol is methanol or ethanol, alternatively methanol. In some embodiments of the invention, the C₁-C₄ alcohol is present in an amount of at least 1.1 equivalents based on equivalents of carbon-carbon unsaturation in the triglyceride or fatty acid methyl ester, alternatively at least 2 equivalents, alternatively at least 5 equivalents, alternatively at least 10 equivalents, alternatively at least 15 equivalents. In one embodiment of the invention, the alcohol is present in an amount of no more than 25 equivalents.

In some embodiments of the invention, the reaction mixture for alcohol addition is heated in a temperature range from 35°C to 70°C for at least 0.5 hours. Alternatively, the temperature is at least 40°C , alternatively at least 45°C, alternatively at least 50°C. Alternatively, the temperature is no greater than 65°C, alternatively no greater than 63°C, alternatively no greater than 60°C, alternatively no greater than 58°C, alternatively no greater than 55°C, alternatively no greater than 53°C, alternatively no greater than 50°C. Alternatively, the reaction time is at least 1 hour, alternatively at least 2 hours, alternatively at least 3 hours. Alternatively, the reaction time is no greater than 10 hours, alternatively no greater than 8 hours, alternatively no greater than 5 hours. When the catalyst is a heterogeneous catalyst, it may be removed from the reaction mixture by filtration, centrifugation, or any other standard method for separating solids and liquids. Excess alcohol may be removed by evaporation, or in the case of a triglyceride starting material, it may be left in the reaction mixture for the transesterification reaction.

In some embodiments of the invention, the triglyceride or fatty acid methyl ester contains, on average, from one to four units of carbon-carbon unsaturation per fatty acid chain; alternatively at least two; alternatively no more than three.

Transesterification of the triglyceride ether with methanol is performed by standard methods known for transesterification of triglycerides. Acidic or basic catalysts may be used, either of which may be a heterogeneous or a homogeneous catalyst. In some embodiments of the invention, the catalyst comprises an acidic or basic ion exchange resin. Glycerol obtained from the transesterification reaction may be removed as part of a separate liquid phase, or by any other suitable separation technique, e.g., centrifugation, distillation. Heterogeneous catalysts may be removed from the reaction mixture by filtration, centrifugation, or any other standard method for separating solids and liquids.

In some embodiments of the invention, the catalyst for transesterification comprises a metal oxide or metal complex immobilized on an ion exchange resin having acid functionality. Preferably, the metal oxide or metal complex is an amphoteric compound. In one embodiment, the metal is Sn, Zn, Ge(II), Cu(II), Ni(II), Fe(II), Fe(III), Al(III), Pt(IV), V(IV) or V(V). Especially preferred metals are Sn, Zn, Ni, Al and Pt as oxides or complexes with other ligands such as sulfides. In one embodiment of the invention, the metal complex is a dialkyl metal oxide, and the alkyl groups in the dialkyl metal oxide are C₁ to C₁₀ alkyl groups, alternatively C₂ to C₄ alkyl groups, alternatively n-butyl groups. A particularly preferred dialkyl metal oxide is dibutyl tin oxide (DBTO). The catalyst is formed by immobilizing the metal oxide and/or dialkyl metal oxide on the ion exchange resin by heating the oxide and the resin together with a solvent. In one embodiment of the invention, the amount of metal oxide is sufficient to complex from 5% to 50% of the acid functionality of the resin, alternatively from 10% to 20%. In one embodiment of the invention, one mole of metal oxide or dialkyl metal oxide complexes two moles of acid groups. For a typical acidic ion exchange resin having from 0.4 to 8 meq/g acid functionality, preferably an amount of metal oxide and/or dialkyl metal oxide from 10% to 50% of the meq/g of total dry resin weight is added, alternatively from 15% to 35%.

## Claims

1. A method for production of stabilized fatty acid methyl esters; said method comprising either:
(a) (i) contacting an unsaturated triglyceride with a C₁-C₄ alcohol and an acidic catalyst to produce a triglyceride ether, and (ii) transesterifying said triglyceride ether with methanol to produce a stabilized fatty acid methyl ester; or
(b) contacting an unsaturated fatty acid methyl ester with a C₁-C₄ alcohol and an acidic catalyst to produce a stabilized fatty acid methyl ester.

2. The method of claim 1 in which the acidic catalyst is an acidic ion exchange resin, and reaction temperature for contacting an unsaturated triglyceride with a C₁-C₄ alcohol or contacting an unsaturated fatty acid methyl ester with a C₁-C₄ alcohol is from 35°C to 70°C.

3. The method of claim 2 in which the acidic ion exchange resin has sulfonic acid functionality.

4. The method of claim 1 in which the C₁-C₄ alcohol is methanol.

5. The method of claim 4 in which the acidic catalyst is an acidic ion exchange resin.

6. The method of claim 5 in which the acidic ion exchange resin has sulfonic acid functionality, and reaction temperature for contacting an unsaturated triglyceride with a C₁-C₄ alcohol or contacting an unsaturated fatty acid methyl ester with a C₁-C₄ alcohol is from 35°C to 70°C.

7. The method of claim 1 in which triglyceride or fatty acid methyl ester contains from one to four units of carbon-carbon unsaturation per fatty acid chain.

8. The method of claim 7 in which the acidic catalyst is an acidic ion exchange resin with sulfonic acid functionality.

9. The method of claim 8 in which the C₁-C₄ alcohol is methanol.

10. The method of claim 9 in which methanol reacts with the unsaturated triglyceride or unsaturated fatty acid methyl ester at a temperature from 35°C to 70°C.
